# EUROPEAN PATENT APPLICATION

(11) **EP 4 516 201 A1**
(43) Date of publication of application: **05.03.2025**
(21) Application number: 24197401.3
(22) Date of filing: 29.08.2024
(51) Int. Cl.: A61B 1/00, A61B 1/012

(54) **CONNECTING DEVICE AND ENDOSCOPE ASSEMBLY**

(30) Priority: 01.09.2023 CN 202311127730
(71) Applicant: Micro-Tech (Nanjing) Co., Ltd., Nanjing, Jiangsu 210032 (CN)
(72) Inventor: Li, Jing, Jiangsu, 210032 (CN); Li, Changqing, Jiangsu, 210032 (CN); Xi, Jiefeng, Jiangsu, 210032 (CN); Shen, Zhenghua, Jiangsu, 210032 (CN); Zhang, Rui, Jiangsu, 210032 (CN); Ding, Xiaoliang, Jiangsu, 210032 (CN); Wang, Qi, Jiangsu, 210032 (CN)
(74) Representative: Ullrich & Naumann PartG mbB

(57) **Abstract**

The present disclosure discloses a connecting device (10) and an endoscope assembly, and belongs to the technical field of medical instruments. A body (100) of the connecting device includes a connecting portion (110) via which a first endoscope (20) may be connected and fixed. A side of the body opposite to the connecting portion is provided with a receiving groove (120) for directly receiving a second endoscope (30) therein to facilitate a connection and fixation of the second endoscope. Meanwhile, a connecting component (130) for engaging the second endoscope is provided on the body, so that the second endoscope may be fixed within the receiving groove, thereby achieving a connection between the first endoscope and the second endoscope, facilitating assembly and disassembly thereof, and also improving a stability of connection, which is convenient for physicians to operate.

## Description

### FIELD OF THE INVENTION

The present disclosure pertains to the field of medical instruments, and in particular is related to a connecting device and an endoscope assembly.

### BACKGROUND OF THE INVENTION

During endoscopic surgeries, especially surgeries related to biliary tract system, physicians may need to use two kinds of endoscopes. For example, during bile duct exploration surgeries, physicians may need to use a duodenoscope and a choledochoscope at the same time.

Generally, when physicians perform these surgeries, they will connect and fix the two endoscopes together to facilitate a single-handed operation. In currently used connection approaches, a binding tape is usually to use to bind the choledochoscope and the duodenoscope together; however, the binding and disassembly process thereof is inconvenient for operation.

### SUMMARY OF THE INVENTION

The objective of the present disclosure is to provide a connecting device for solving the technical problem of inconvenient operation in current endoscope connection approaches; another objective of the present disclosure is to provide an endoscope assembly.

Technical solution: the present disclosure discloses a connecting device for connecting a first endoscope and a second endoscope, including:
a body including a connecting portion for connecting the first endoscope, wherein the body is further provided with a receiving groove that is configured to face away from the connecting portion to receive the second endoscope;
a connecting component provided on the body, wherein the connecting component is configured to connect the second endoscope so that the second endoscope may be fixed in the receiving groove.

According to an aspect of the present disclosure, a direction extending from the receiving groove towards the connecting portion is defined as a first direction, an extending direction of the receiving groove is defined as a second direction, with the first direction intersecting with the second direction;
wherein the connecting component forms a first orthographic projection on a plane perpendicular to the first direction, and the body forms a second orthographic projection on this plane; wherein at least a portion of the first orthographic projection falls outside the second orthographic projection, and is located further away in the second direction with respect to the second orthographic projection.

According to an aspect of the present disclosure, the connecting component includes:
a hook portion connected to the body, wherein at least a portion of the hook portion is located further away in the second direction with respect to the body, and the second direction is defined as the extension direction of the receiving groove;
the hook portion is configured to hook on the second endoscope for connecting with the second endoscope.

According to an aspect of the present disclosure, the second endoscope includes an endoscope body and a clip entrance connected with each other, wherein the clip entrance protrudes from the endoscope body, and the hook portion is configured to at least partially hook on the clip entrance.

According to an aspect of the present disclosure, the hook portion is configured to wind around the clip entrance.

According to an aspect of the present disclosure, the hook portion includes:
an extension arm connected to the body, wherein at least a portion of the extension arm is located on a side of the body that extends further away in the second direction;
a clamping lug which is opposite to and spaced apart from the extension arm;
an abutting arm which is connected between the extension arm and the clamping lug;
wherein the abutting arm is configured for contacting with a top surface of the clip entrance, and the extension arm and the clamping lug are respectively configured for contacting with two opposite entrance side faces of the clip entrance, such that the hook portion may hook on the clip entrance; wherein the two entrance side faces are respectively connected to the top surface of the clip entrance.

According to an aspect of the present disclosure, the extension arm includes:
a first surface that extends away from the connecting portion in the first direction, and is configured for abutting against a side surface of the endoscope body that faces towards the body;
a second surface that is connected to the first surface at an end away from the body, and is configured for abutting against a side face of the clip entrance that faces towards the body;
wherein the first direction is a direction extending from the receiving groove towards the connecting portion.

According to an aspect of the present disclosure, the connecting component includes:
a winding portion connected to the body, wherein at least a portion of the winding portion is provided at one side of the body that extends further away in the second direction, said second direction being an extension direction of the receiving groove;
wherein the winding portion is configured for winding around the second endoscope so as to connect with the second endoscope.

According to an aspect of the present disclosure, the direction extending from the receiving groove towards the connecting portion is defined as a first direction, the extension direction of the receiving groove is defined as a second direction, and the body has a third direction, the first direction, the second direction and the third direction intersecting with each other;
the connecting component forms a first orthographic projection on a plane perpendicular to the first direction, the body forms a second orthographic projection on this plane, wherein at least parts of the first orthographic projection fall outside the second orthographic projection, and are symmetrically distributed at two sides of the second orthographic projection with respect to the third direction.

According to an aspect of the present disclosure, the connecting component includes:
a snapping portion that is connected on one side of the body opposite to the connecting portion and is provided on both sides of the receiving groove with respect to a third direction; a direction extending from the receiving groove to the connecting portion is defined as a first direction, an extending direction of the receiving groove is defined as the second direction, with the first direction, the second direction and the third direction intersecting with each other;
wherein the snapping portion is configured to clamp on both sides of the second endoscope such that the second endoscope may be fixed within the receiving groove.

According to an aspect of the present disclosure, the snapping portion forms an opening communicated with the receiving groove, and the snapping portion is so configured that the second endoscope may be received into the receiving groove via the opening;
wherein the opening has a maximum dimension D in the third direction, and a portion of the second endoscope that is accommodated within the receiving groove has a maximum size L, wherein D≤L.

According to an aspect of the present disclosure, the snapping portion includes:
at least two side wings that are oppositely arranged with respect to the third direction, wherein the at least two side wings are connected to the body and are respectively provided at two side edges of the receiving groove with respect to the third direction.

According to an aspect of the present disclosure, the side wing include:
a main body that connected to the body and is provided with a fitting slot; and
a rotating body that is rotatably connected to the main body and is arranged in the fitting slot,
wherein the rotating body is configured for contacting the second endoscopes in a rotatable way to guide the second endoscope into the receiving groove.

According to an aspect of the present disclosure, the rotating body is provided with a plurality of anti-slip recesses, wherein the plurality of anti-slip recesses is spaced apart along an outer circumferential surface of the rotating body.

According to an aspect of the present disclosure, the rotating body is made of elastic materials.

According to an aspect of the present disclosure, a plurality of the snapping portions is provided and are spaced apart in the second direction.

According to an aspect of the present disclosure, a minimum spacing between at least two side wings in one snapping portions is different from a minimum spacing between at least two side wings in another snapping portion.

According to an aspect of the present disclosure, the connecting component includes:
a protrusion that is connected to the body and is arranged in the receiving groove, wherein the protrusion is configured to protrude with respect to an inner wall of the receiving groove;
wherein the protrusion is configured to contact with the second endoscope when the second endoscope is received within the receiving groove, wherein a contact surface between the protrusion and the second endoscope is a rough surface.

According to an aspect of the present disclosure, the protrusion is made of elastic materials.

According to an aspect of the present disclosure, a plurality of protrusions is provided in a spaced manner in the second direction, wherein the second direction is the extension direction of the receiving groove.

According to an aspect of the present disclosure, the connecting component includes:
a bonding portion that is connected to the body and is adhered to at least a portion of an inner wall of the receiving groove;
the bonding portion is configured for bonding the second endoscope so that the second endoscope may be fixed within the receiving groove.

According to an aspect of the present disclosure, a plurality of bonding portions spaced apart from each other is provided.

According to an aspect of the present disclosure, the body and the connecting component are of an integrally formed structure.

The present disclosure correspondingly discloses an endoscope assembly that includes:
the connecting device as described above; and
a first endoscope connected to the connecting portion.

According to an aspect of the present disclosure, the connecting device and the first endoscope are of an integrally formed structure.

According to an aspect of the present disclosure, the endoscope assembly further includes:
a second endoscope that is received in the receiving groove of the connecting device, and is connected with the connecting component of the connecting device so as to be fixed within the receiving groove.

According to an aspect of the present disclosure, the second endoscope includes:
an endoscope body that is received within the receiving groove;
a clip entrance provided on the endoscope body;
wherein a hook portion of the connecting component hooks on the clip entrance to connect with the second endoscope.

According to an aspect of the present disclosure, the second endoscope includes:
an endoscope body that is received within the receiving groove and engages with the snapping portion of the connecting component in a snapping way.

According to an aspect of the present disclosure, the second endoscope includes:
an endoscope body received in the receiving groove;
wherein a bonding portion of the connecting component is bonded to the second endoscope so that the second endoscope can be fixed within the receiving groove.

According to an aspect of the present disclosure, the second endoscope includes:
an endoscope body received in the receiving groove;
wherein a winding portion of the connecting component is configured to wind around the second endoscope so as to be connected to the second endoscope.

Beneficial effect: compared with the prior art, the connecting device of the embodiments of the present disclosure is configured for connecting the first endoscope and the second endoscope, and includes: a body including a connecting portion for connecting the first endoscope, wherein the body has a receiving groove facing away from the connecting portion to receive the second endoscope; a connecting component provided on the body and configured to connect to the second endoscope so that the second endoscope can be fixed in the receiving groove. The body of the connecting device includes a connecting portion, and the first endoscope can be connected and fixed by the connecting portion. The side of the body facing away from/opposite to the connecting portion is provided with a receiving groove, so that the second endoscope can be directly received within the receiving groove, so as to facilitate a connection and fixation of the second endoscope. Meanwhile, a connecting component is provided on the body, and the second endoscope is connected by the connecting component, so that the second endoscope can be fixed in the receiving groove. By this, a connection between the first endoscope and the second endoscope may be realized, and it is convenient for assembly and disassembly; meanwhile, it may also improve a stability of the connection, and is convenient for physicians to operate.

Compared with the prior art, the endoscope assembly of the present disclosure includes: the connecting device as described above, and a first endoscope connected to the connecting portion. The endoscope assembly may include all the technical features and technical effects of the connecting device described above, which will not be described in detail here.

### BRIEF DESCRIPTION OF THE DRAWINGS

In order to more clearly explain the technical solutions of the embodiments in the present disclosure, the drawings used in the description of the embodiments will be briefly introduced below. Obviously, the drawings described below merely illustrate some embodiments of the present disclosure. For those skilled in the art, other drawings can be obtained based on these drawings without creative work.
FIG. 1 is a schematic structural diagram of a connecting device provided in a first embodiment of the present disclosure;
FIG. 2 is a schematic structural diagram from a first perspective illustrating that the connecting device according to the first embodiment of the present disclosure is used to connect a first endoscope and a second endoscope;
FIG. 3 is a schematic structural diagram illustrating that the second endoscope schematically illustrated in FIG. 2 is detached from the connecting device;
FIG. 4 is a schematic structural diagram from a second perspective illustrating that the connecting device according to the first embodiment of the present disclosure is used to connect the first endoscope and the second endoscope;
FIG. 5 is a schematic structural diagram illustrating that the second endoscope schematically illustrated in FIG. 4 is detached from the connecting device;
FIG. 6 is an exploded schematic diagram of the parts schematically illustrated in FIG. 4;
FIG. 7 is a schematic structural diagram from a first perspective of a connecting device provided in a second embodiment of the present disclosure;
FIG. 8 is a schematic structural diagram from a second perspective of the connecting device provided in the second embodiment of the present disclosure;
FIG. 9 is a schematic structural diagram from a first perspective illustrating that the connecting device according to the second embodiment of the present disclosure is used to connect a first endoscope and a second endoscope;
FIG. 10 is a schematic structural diagram illustrating that the second endoscope schematically illustrated in FIG. 9 is detached from the connecting device;
FIG. 11 is an exploded schematic diagram of the parts schematically illustrated in FIG. 9;
FIG. 12 is a schematic structural diagram of a connecting device provided in a third embodiment of the present disclosure;
FIG. 13 is a partially enlarged schematic structural diagram illustrating an area A of the connecting device schematically illustrated in FIG. 12;
FIG. 14 is a schematic structural diagram from a first perspective illustrating that the connecting device according to the third embodiment of the present disclosure is used to connect a first endoscope and a second endoscope;
FIG. 15 is a schematic structural diagram illustrating that the second endoscope schematically illustrated in FIG. 14 is detached from the connecting device;
FIG. 16 is an exploded schematic diagram of the parts schematically illustrated in FIG. 14;
FIG. 17 is a schematic structural diagram of a connecting device provided in a fourth embodiment of the present disclosure;
FIG. 18 is a schematic structural diagram from a first perspective illustrating that the connecting device according to the fourth embodiment of the present disclosure is used to connect a first endoscope and a second endoscope;
FIG. 19 is a schematic structural diagram illustrating that the second endoscope schematically illustrated in FIG. 18 is detached from the connecting device;
FIG. 20 is an exploded schematic diagram of the parts schematically illustrated in FIG. 18;
FIG. 21 is a schematic structural diagram of a connecting device provided in a fifth embodiment of the present disclosure;
FIG. 22 is a schematic structural diagram illustrating that a connecting device according to a sixth embodiment of the present disclosure is used to connect a first endoscope and a second endoscope;
FIG. 23 is a cross-sectional view along line A-A in FIG. 22;
FIG. 24 is a schematic structural diagram of a connecting device provided in a seventh embodiment of the present disclosure;
FIG. 25 is a schematic structural diagram illustrating that a connecting device according to an eighth embodiment of the present disclosure is used to connect a first endoscope and a second endoscope;
FIG. 26 is a schematic structural diagram illustrating that the second endoscope schematically illustrated in FIG. 25 is detached from the connecting device.

Reference signs: 10-connecting device; 100-body; 110-connecting portion; 120-receiving groove; 121-inner wall; 130-connecting component; 140-hook portion; 141-extension arm; 1411-first surface; 1412-second surface; 142-clamping lug; 143-abutting arm; 150-snapping portion; 151-side wing; 1511-main body; 1512-rotating body; 1513-anti-slip recess; 1514-fitting slot; 152-opening; 160-protrusion; 161-rough surface; 170-bonding portion; 180-winding portion; 20-first endoscope; 30-second endoscope; 300 -endoscope body; 310-clip entrance; 311-top surface; 312-entrance side face.

### DETAILED DESCRIPTION OF THE EMBODIMENTS

The technical solutions in the embodiments of the present disclosure will be described clearly and completely below in conjunction with the drawings of the embodiments of the present disclosure. Obviously, the described embodiments merely illustrate some embodiments of the present disclosure, rather than all of the embodiments. Based on the embodiments in the present disclosure, all other embodiments obtained by those skilled in the art without creative work are within the scope of protection of the present disclosure.

In the description of the present disclosure, it should be understood that the terms" proximal end" and "distal end" are defined with reference to the surgical operator (physician), wherein the "proximal end" is an end of the surgical tool closer to the operator, and the "distal end" is an end farther away from the operator with respect to the "proximal end", that is, the end closer to the patient. The orientation or position relationship indicated by the terms "inside", "outside", "upper", "lower", "left", "right", etc. is based on the orientation or position relationship shown in the accompanying drawings, and is only used for a convenient and simplified description of the present disclosure, rather than indicating or implying that the device or element referenced must have a specific orientation, be constructed and operated in a specific orientation, and therefore cannot be understood as a limitation on the present disclosure. In the description of the present disclosure, the term "multiple" means two or more members, while the term "at least one" means one, two or more members, unless otherwise clearly and specifically defined. The term "connection" may mean a direct connection, that is, one object is directly connected to another object in a contacted manner, or may mean an indirect connection, that is, one object is connected to another object via other intermediate obj ects.

It should also be noted that in the drawings of the specification of this disclosure, an arrow marked X is used to indicate a first direction X, an arrow marked Y is used to indicate a second direction Y, and an arrow marked Z is used to indicate a third direction. The first direction X, the second direction Y, and the third direction Z are introduced to more clearly describe the structure and relative positional relationship of each member of the connecting device 10 according to the embodiment of the present disclosure. It should be understood that the first direction X, the second direction Y, and the third direction Z are relative directions defined based on relative positions between respective members, rather than absolute orientations. When the connecting device 10 is actually used, the first direction X, the second direction Y and the third direction Z can be directed to any direction in space, as long as it is satisfied that the first direction X, the second direction Y and the third direction Z intersect with each other. Preferably, the first direction X, the second direction Y and the third direction Z are orthogonal to each other.

The applicant noted that during some endoscopic surgeries, physicians may need to use two kinds of endoscopes. For example, during bile duct exploration surgeries, physicians may need to use a duodenoscope and a choledochoscope at the same time. First, the duodenoscope is applied to enter the duodenum, and then the choledochoscope is guided by the duodenoscope into the bile duct system. In this way, stones in the bile duct may be directly observed and treated under the guidance of the endoscope. In these surgical operations, it is obviously inconvenient for a physician to hold two endoscopes at the same time, thus he tends to connect and fix the two endoscopes together to facilitate a single-handed operation. In currently used connection approaches, a binding tape is usually to use to bind the choledochoscope and the duodenoscope together; however, the binding and disassembly process thereof is inconvenient for operation.

In view of this, an embodiment of the present disclosure provides a connecting device 10 for attaching both the first endoscope 20 and the second endoscope 30, which can solve at least one of the above technical problems.

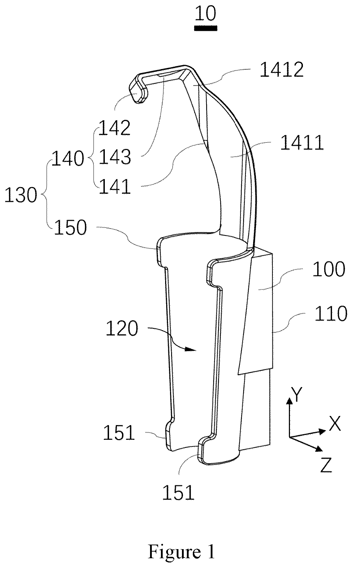

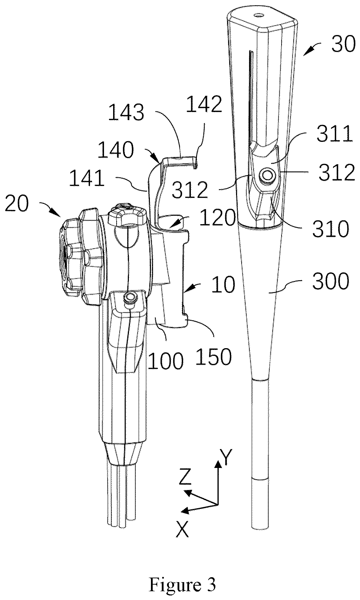

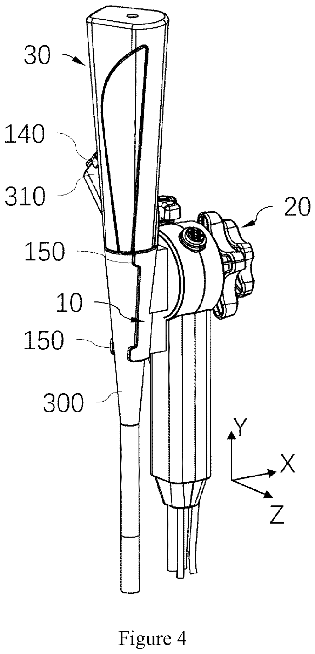

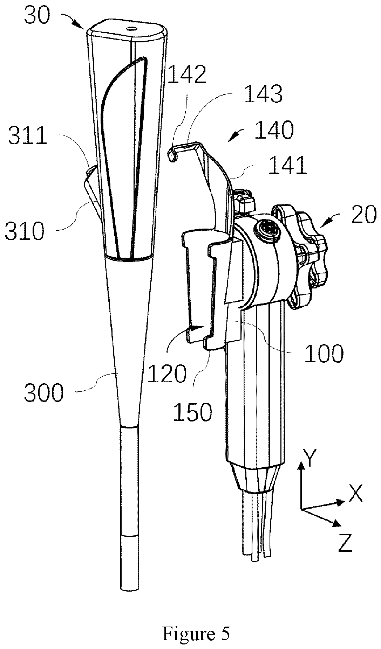

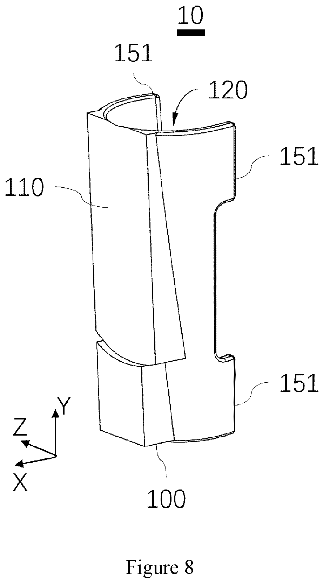

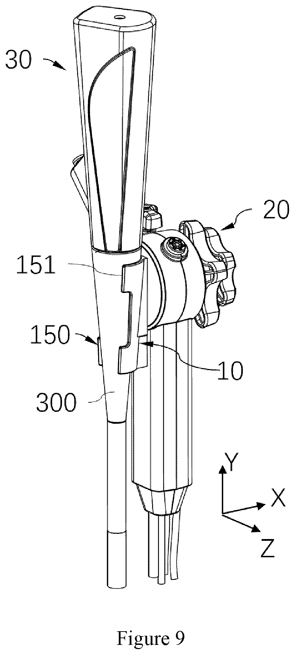

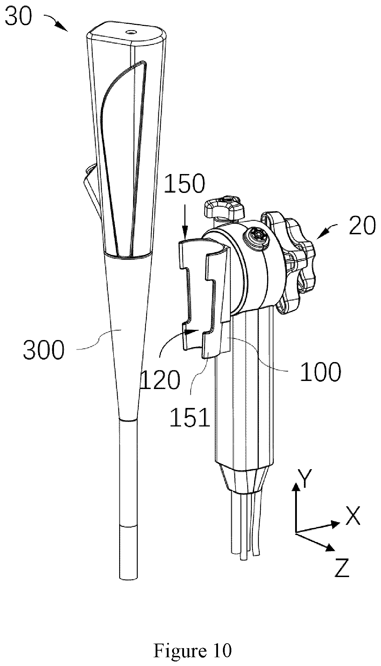

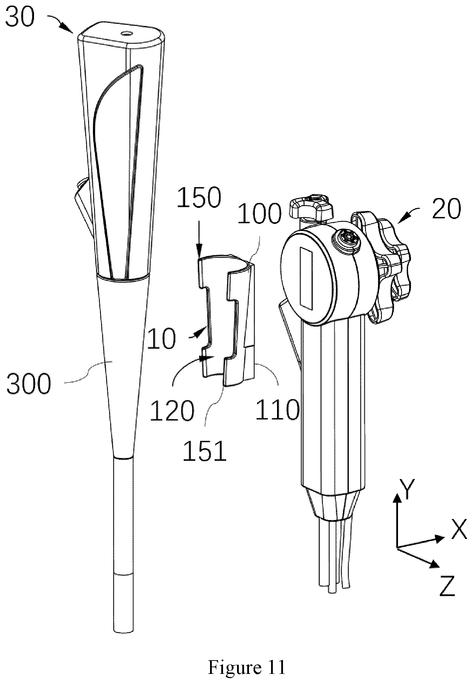

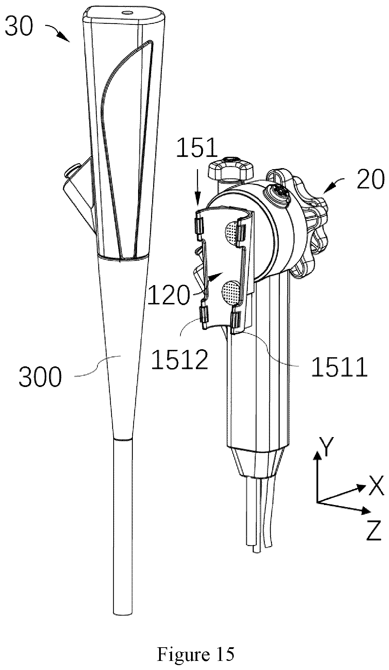

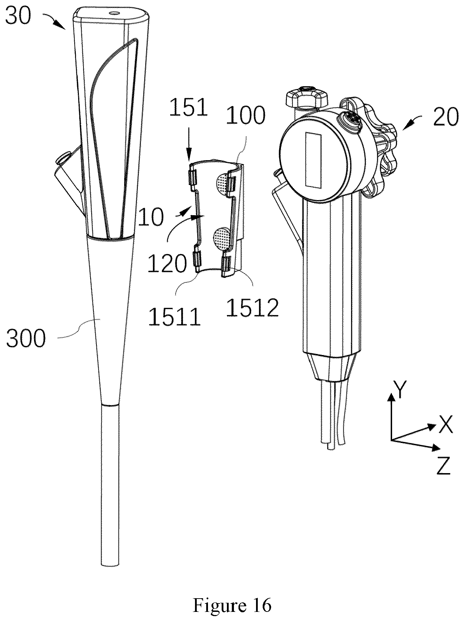

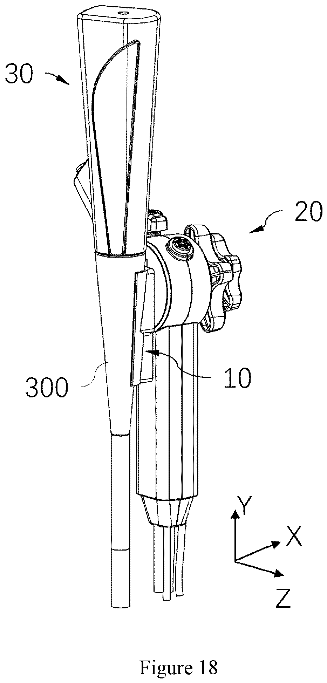

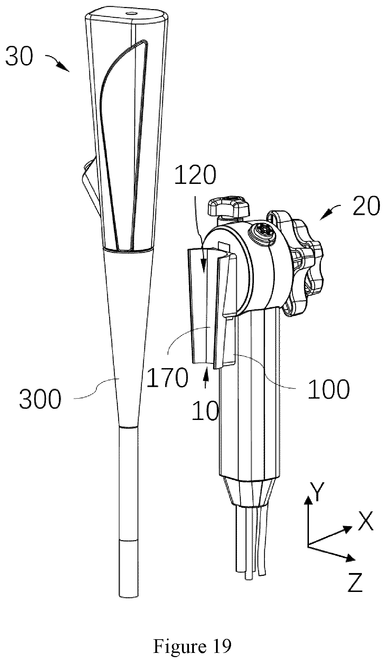

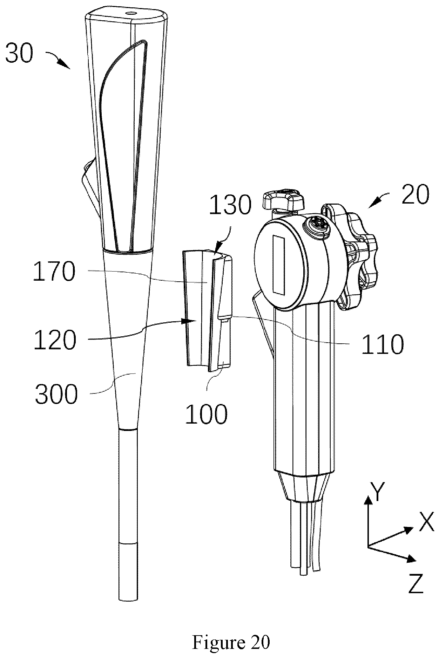

Referring to FIGS. 1 to 20 together, the connecting device 10 provided in the embodiment of the present disclosure comprises a body 100 and a connecting component 130. The body 100 comprises a connecting portion 110 for connecting the first endoscope 20. The body 100 has a receiving groove 120 facing away from the connecting portion 110. The receiving groove 120 is designed for receiving the second endoscope 30. The connecting component 130 is provided on the body 100 and is used for connecting the second endoscope 30 so that the second endoscope 30 can be fixed in the receiving groove 120. Therefore, the body 100 can be connected between the first endoscope 20 and the second endoscope 30, connect and fix the first endoscope 20 via its connecting portion 110, receive the second endoscope 30 via its receiving groove 120, and fix the second endoscope 30 via a cooperation with the connecting component 130. In this way, the first endoscope 20 and the second endoscope 30 can be stably connected and fixed together, which is beneficial for improving the surgical quality and efficiency.

Referring to FIGS. 1, 7, 8, 12 and 17 together, in some embodiments, the body 100 is an integrally formed structure, that is, the body 100 is an integral structure without splicing. By designing the body 100 as an integrally formed structure, the integrity of the structure is guaranteed, the stability of the structure is improved, and the stability of the connection between the first endoscope 20 and the second endoscope 30 is in turn improved to a certain extent.

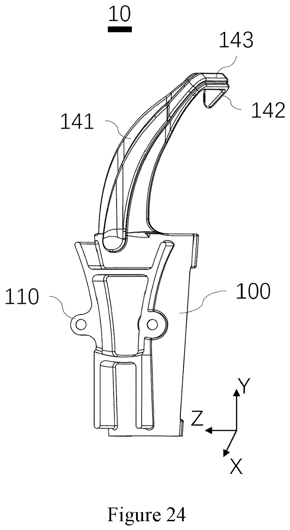

The connecting portion 110 may be formed on one side of the body 100, and this side may be used as a connecting surface for connecting the first endoscope 20 by gluing, welding, screwing, etc., so as to realize a connection and fixation with the first endoscope 20. Alternatively, the connecting portion 110 may be a slot or buckle formed on one side of the body 100, and the first endoscope 20 may be provided with a structure matching with the slot or buckle; by this, a connection and fixation of the first endoscope 20 may be realized by fitting/coupling of matching structures of the connecting portion 110 with the first endoscope 20. Exemplarily, referring to FIG. 24, in the seventh embodiment provided in the present disclosure, the connecting portion 110 may be designed as a screw hole provided on the body 100, and the body 100 and the first endoscope 20 may be connected and fixed together by screws. The number and position of the screw holes may be specifically provided according to actual needs, for example, two or more screw holes may be provided to improve connection stability; for example, the screw holes may be arranged at the edge of the body 100, or they may be arranged in a way penetrating through the body 100. In particular, the screw holes may be communicated with the receiving groove 120, so that a screw may be installed/screwed from the receiving groove 120 into the screw hole for connecting the connecting device 10 and the first endoscope 20 together.

The receiving groove 120 is formed on the other side, opposite to the connecting portion 110, of the body 100. At least a portion of this side is configured to take a curved shape or other recessed shapes to form the receiving groove 120; that is, at least a portion of this side forms an inner wall 121 for the receiving groove 120. The second endoscope 30 can be accommodated/received in the receiving groove 120. The inner wall 121 of the receiving groove 120 is configured to match with the endoscope body 300 of the second endoscope 30, such that a surface of the endoscope body 300 of the second endoscope 30 abuts snugly against the inner wall 121 of the receiving groove 120, therefore the second endoscope 30 can be stably received in the receiving groove 120.

Further, in some embodiments, the body 100 and the connecting component 130 are of an integrally formed structure, which can ensure a structural integrity for the connecting device 10 and improve the stability of the structure, thereby further improving the connection stability of the first endoscope 20 and the second endoscope 30.

Referring to FIG. 1, in some embodiments, a direction extending from the receiving groove 120 to the connecting portion 110 is defined as the first direction X, and the extension direction of the receiving groove 120 is defined as the second direction Y, wherein the first direction X intersects with the second direction Y The connecting component 130 forms a first orthographic projection on a plane perpendicular to the first direction X, and the body 100 forms a second orthographic projection on this plane. At least a portion of the first orthographic projection falls outside the second orthographic projection, and is located further away in the second direction Y with respect to the second orthographic projection. It is to be noted that in the description of the present disclosure, a member being located further away in a certain direction with respect to another member means that the member is located at one end of the other member in the certain direction. It is also to be noted that said plane is a projection/virtual plane, and is not physically provided in the structure of the connecting device 10. When a relative positional relationship between the region of the first orthographic projection and the region of the second orthographic projection is subsequently verified, a flat planar structure perpendicular to the first direction X can be provided as a projection plane. The connecting component 130 and the body 100 may be respectively irradiated with lights parallel to the first direction X, and the projections respectively formed on the projection plane are their corresponding orthographic projections, namely the first orthographic projection and the second orthographic projection.

Among which, at least a portion of the first orthographic projection is located outside the second orthographic projection, and at least a portion of the first orthographic projection is located further away in the second direction Y with respect to the second orthographic projection. That is to say, with respect to the body 100, at least a portion of the connecting component 130 is arranged on a side of the body 100 that extends further away in the second direction Y By this, in a region extending further from the receiving groove 120, a force acting on the second endoscope 30 can be provided by the connecting component 130, so that the second endoscope 30 may be connected more stably.

Specifically, referring to FIGS. 1 to 6 again, in the first embodiment, the connecting component 130 comprises a hook portion 140 that is connected to the body 100, and at least a portion of hook portion 140 is located further away in the second direction Y with respect to the body 100. The hook portion 140 is designed for hooking on the second endoscope 30 and for coupling with the second endoscope 30. Therefore, when the second endoscope 30 is received in the receiving groove 120, it may be caught by hooking on the second endoscope 30 via the hook portion 140, thereby ensuring a connection stability of the second endoscope 30; by this, the second endoscope 30 may be more stably received in the receiving groove 120. Moreover, the orthographic projection of the hook portion 140 on a plane perpendicular to the second direction Y is located further away in the second direction Y with respect to the second orthographic projection of the body 100. By this, the hook portion 140 may provide a force in a region extending further from the receiving groove 120 (i.e., extending further in the second direction Y), such that the second endoscope 30 may be connected more stably.

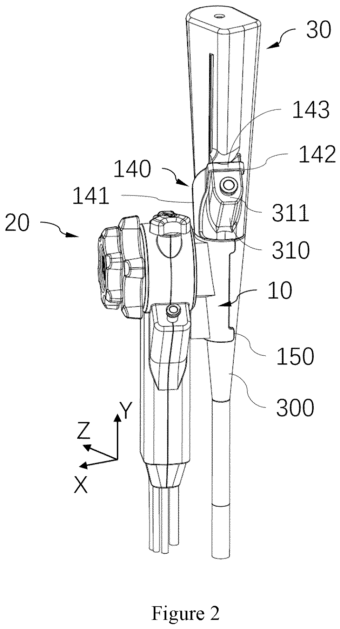

Referring to FIGS. 2, 3, 4, 5 and 6 together, in the first embodiment, the second endoscope 30 comprises an endoscope body 300 and a clip entrance 310, and the hook portion 140 is provided to at least partially hook on the clip entrance 310. The clip entrance 310 is a protruding structure provided on the endoscope body 300 of the endoscope, which usually protrudes from the endoscope body 300 and has a clip inlet formed thereon. During a surgery, a physician can insert corresponding surgical instrument through the clip inlet. In this embodiment, the hook portion 140 is hooking on the clip entrance 310, and the protruding structure of the clip entrance 310 can be used to support the hook portion 140; thus, the connection stability may be improved by a force generated therebetween, so that it is further ensured that the second endoscope 30 is firmly connected to the connecting device 10.

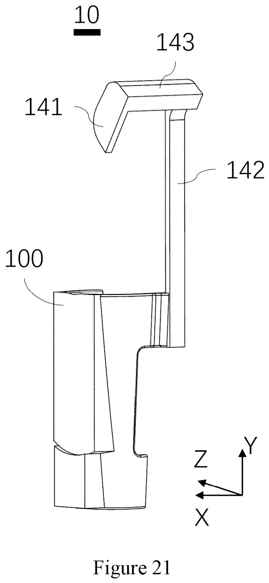

Further, referring to FIG. 2, 3, 5, 6 and 21 together, in the first and fifth embodiments, the hook portion 140 comprises an extension arm 141, a clamping lug 142 and an abutting arm 143. The extension arm 141 is connected to the body 100 and is at least partially located on the side of the body 100 that extends further away in the second direction Y; the clamping lug 142 is opposite to and is spaced apart from the extension arm 141; the abutting arm 143 is connected between the extension arm 141 and the clamping lug 142. Among which, the abutting arm 143 is designed for contacting with a top surface 311 of the clip entrance 310, and the extension arm 141 and the clamping lug 142 are respectively designed for contacting with the two opposite entrance side faces 312 of the clip entrance 310; by this, the hook portion 140 is hooking on the clip entrance 310, wherein the two entrance side faces 312 are respectively connected to the top surface of the clip entrance 310. That is to say, the hook portion 140 has a bending structure, and the extension arm 141, the abutting arm 143 and the clamping lug 142 are formed in a bent configuration in sequence from the edge of the body 100. The extension arm 141 contacts one side face of the clip entrance 310, the clamping lug 142 contacts the other side face of the clip entrance 310, and the abutting arm 143 contacts/abuts against the top surface 311of the clip entrance 310, so that the top surface 311 may function to support the connecting device 10. This design, cooperating with the receiving groove 120, makes the connection between the connecting device 10 and the second endoscope 30 more stable. Specifically, referring to FIG. 2, in the first embodiment, the clamping lug 142 abuts against one of the entrance side faces 312 of the clip entrance 310 that is away from the body 100, so that the hook portion 140 may provide a force to the second endoscope 30 to pull/press the second endoscope 30 towards the body 100; by this, the connection stability between the connecting device 10 and the second endoscope 30 may be improved further. Specifically, referring to FIG. 21, in the fifth embodiment, the extension arm 141 exerts a pulling force on the clip entrance 310 on the side of the clip entrance 310 that is away from the body 100, so that the connection stability between the connecting device 10 and the second endoscope 30 may be improved further as well.

Further, referring to FIGS. 1 and 2 again, in the first embodiment, the extension arm 141 comprises a first surface 1411 and a second surface 1412. The first surface 1411 extends away from the connecting portion 110 in the first direction X, and is designed for abutting against a side surface of the endoscope body 300 that faces towards the body 100. The second surface 1412 is connected to the first surface 1411 at an end away from the body 100, and is designed for abutting against a side face of the clip entrance 310 that faces towards the body 100. Thereby, the extension arm 141, by its first surface 1411 and second surface 1412, can abutting against the endoscope body 300 and the clip entrance 310 of the second endoscope 30. By this, a connection therebetween may be more tight, and the connection stability between the connecting device 10 and the second endoscope 30 is further improved.

In some embodiments, the hook portion 140 is winded around the clip entrance 310 to interacts with the clip entrance 310, thus improving a connection stability between the connecting device 10 and the second endoscope 30.

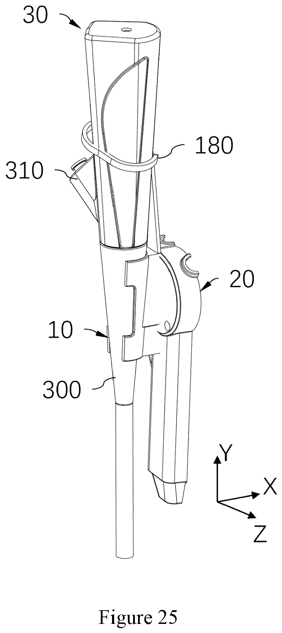

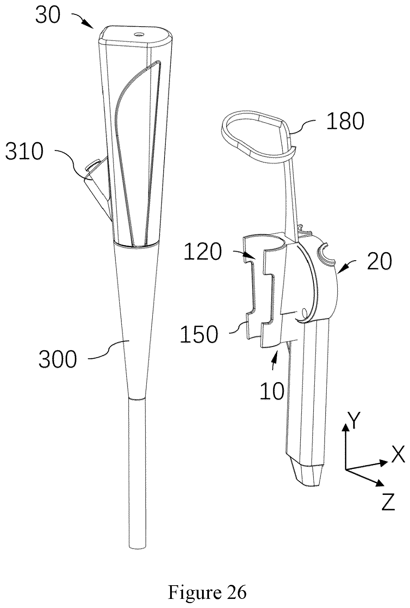

Referring to FIGS. 25 and 26, in an eighth embodiment, the connecting component 130 comprises a winding portion 180 that is connected to the body 100. At least a portion of the winding portion 180 is located on one side of the body 100 that extends further away in the second direction Y (the extension direction of the receiving groove 120). The winding portion 180 is designed for winding around the second endoscope 30 to connect with the second endoscope 30. It can be understood that in this embodiment, a structure of the winding portion 180 of the connecting component 130 is similar to that of the hook portion 140 in the above embodiment, in that both of them are partially located on one side of the body 100 that extends further away from the receiving groove 120, and both are associated with the second endoscope 30. The difference is that the winding portion 180 is wound around the second endoscope 30 to achieve a connection with the second endoscope 30. Specifically, the winding portion 180 can be wound around the endoscope body 300 of the second endoscope 30, by which the connecting component 130 can be stably connected to the second endoscope 30. By this, the second endoscope 30 may be fastened more stably in the receiving groove 120, thus improving the connection stability between the connecting device 10 and the second endoscope 30.

In some embodiments, the direction extending from the receiving groove 120 towards the connecting portion 110 is defined as the first direction X, and the extension direction of the receiving groove 120 is defined as the second direction Y The body 100 has a third direction Z that intersects the first direction X, the second direction Y with each other. The connecting component 130 forms a first orthographic projection on a plane perpendicular to the first direction X, and the body 100 forms a second orthographic projection on this plane. At least parts of the first orthographic projection fall outside the second orthographic projection, and are symmetrically distributed at two sides of the second orthographic projection with respect to the third direction Z. That is to say, with respect to the body 100, at least parts of the connecting component 130 are provided, regarding to the third direction Z, at two sides of the body 100. By this, forces acting on the second endoscope 30 may be provided by the connecting component 130 on both sides of the receiving groove 120 in the third direction Z, which enables the second endoscope 30 to be connected more stably.

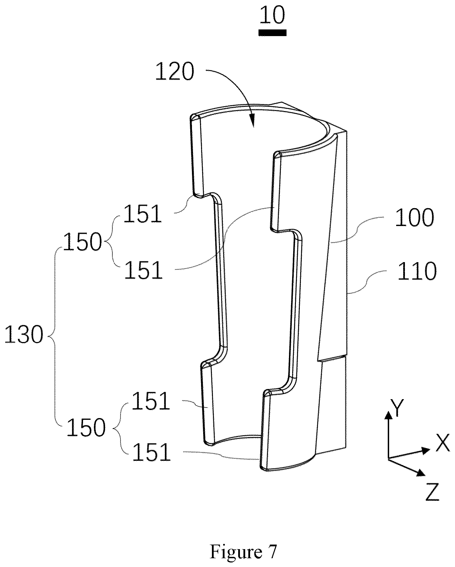

Specifically, referring to FIGS. 7 to 11 and 22, in the second embodiment and the sixth embodiment, the connecting component 130 comprises a snapping portion 150, which is connected on a side of the body 100 opposite to the connecting portion 110 and is provided on both sides of the receiving groove 120 regarding to the third direction Z. Among which, a direction extending from the receiving groove 120 to the connecting portion 110 is defined as the first direction X, the extending direction of the receiving groove 120 is defined as the second direction Y, and the first direction X, the second direction Y and the third direction Z intersect with each other.

Among which, the snapping portion 150 is designed for clamping on both sides of the second endoscope 30 so that the second endoscope 30 can be fixed within the receiving groove 120. Snapping portions 150 may be provided on edges of the receiving groove 120 at both sides, and may clamp/snap on the second endoscope 30 that is received within the receiving groove 120. By this, the second endoscope 130 can be well confined within the receiving groove 120 to achieve fixation and ensure a connection stability of the connecting device with the second endoscope 30. Specifically, in the second embodiment, the snapping portion 150 comprises at least two side wings 151 that are oppositely arranged regarding to the third direction Z. The at least two side wings 151 are both connected with the body 100, and are respectively provided at the two side edges of the receiving groove 120 with respect to the third direction Z. By providing side wings 151 respectively on both sides of the receiving groove 120, the second endoscope 30 received within the receiving groove 120 may be well limited/confined by the two side wings 151, which can effectively prevent the second endoscope 30 from getting detached/falling off from the receiving groove 120 during surgery.

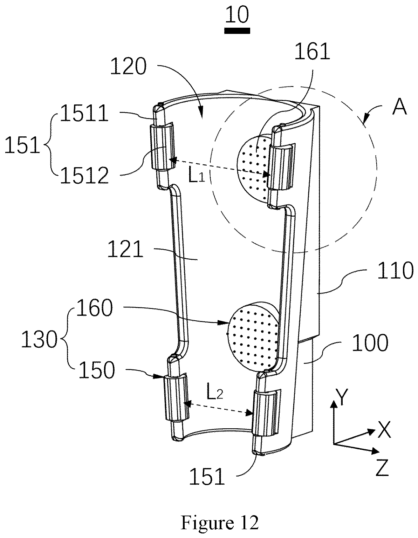

Further, referring to FIGS. 12 to 16 together, similar to the second embodiment, in the third embodiment, the connecting component 130 also comprises a snapping portion 150, and the snapping portion 150 also comprises at least two side wings 151. The difference is that in the third embodiment, each side wing 151 comprises a main body 1511 and a rotating body 1512. Among which, the main body 1511 is connected to the body 100, and is provided with a fitting slot 1514. The rotating body 1512 is rotatably connected to the main body 1511, and is arranged in the fitting slot 1514. The rotating body 1512 is designed for contacting the second endoscopes 30 in a rotatable way, so as to guide the second endoscope 30 into the receiving groove 120. By the rotating body 1512 of the side wings 151, the second endoscope 30 may be conveniently snapped from the snapping portion 150 and received into the receiving groove 120, so as to facilitate an assembly/disassembly of the second endoscope 30 onto/from the connecting device 10 by a physician and to improve operating efficiency.

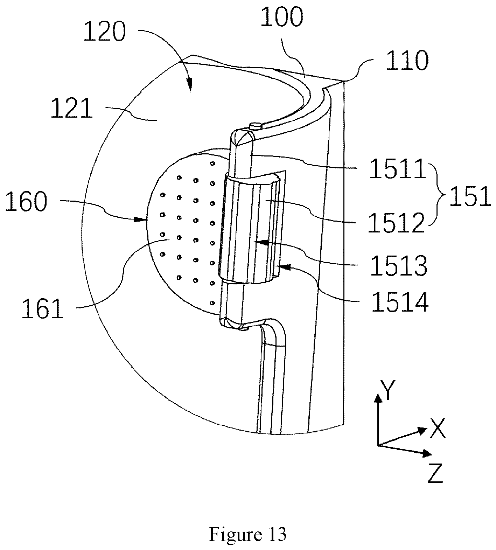

Further, referring to FIG. 13 again, in the third embodiment, the rotating body 1512 is provided with a plurality of anti-slip recesses 1513 spaced apart along an outer circumferential surface of the rotating body 1512. By provision of the anti-slip recesses 1513 on the outer circumferential surface of the rotating body 1512, a roughness of the outer circumferential surface of the rotating body 1512 may be improved. Thus, during assembly/disassembly of the connecting device 10 and the second endoscope 30, the rotating body 1512 is in contact with the second endoscope 30, and rotates as it is loaded onto or unloaded from the connecting device 10. By this, the connecting device 10 and the second endoscope 30 may be smoothly assembled/disassembled, and the assembly/disassembly efficiency may be improved.

Further, in the third embodiment, the rotating body 1512 is made of elastic materials. Thus, it may be elastically deformed when being pressed by the second endoscope 30. By this, on the one hand, the second endoscope 30 will not be over-pressed, thus preventing the second endoscope 30 from being crushed and damaged, and on the other hand, the adaptability of products may be improved by providing elastic deformation to adapt to second endoscopes 30 of different sizes. Of course, in some embodiments, the rotating body 1512 may not be provided with the anti-slip recess 1513. Still further, the rotating body 1512 may also be made of rigid materials to reduce processing difficulty and cost.

In some embodiments, multiple snapping portions 150 are provided and spaced apart from each other in the second direction Y By providing multiple snapping portions 150, the second endoscope 30 can be fixed at different positions in the extending direction of the receiving groove 120, thereby further improving the fixing effect of the second endoscope 30 and guaranteeing the connection effect between the second endoscope 30 and the connecting device 10.

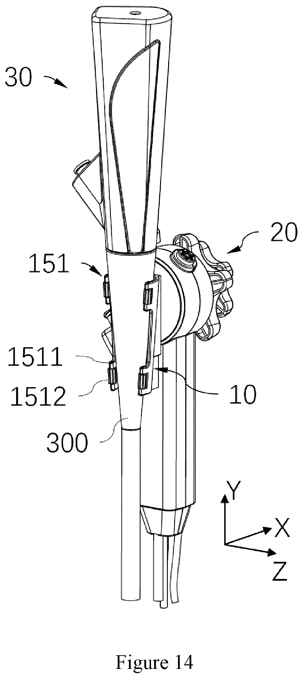

Further, when multiple snapping portions 150 are provided, a minimum distance between at least two side wings 151 for one snapping portion 150 is different from a minimum distance between at least two side wings 151 for another snapping portion 150. Therefore, a size variation of the endoscope body 300 of the second endoscope 30 can be adapted to, so that the endoscope body 300 of the second endoscope 30 can be more securely combined to the connecting device 10, and the connection effect is better. For example, in FIG. 12, double arrow dotted lines are used to mark a minimum distance between two side wings 151 of the upper snapping portion 150 and a minimum distance between two side wings 151 of the lower snapping portions 150 respectively. Among which, the minimum distance between the two side wings 151 of the upper snapping portion 150 is L₁, the minimum distance between the two side wings 151 of the lower snapping portion 150 is L₂, where L₁> L₂. By this, a size gradient from top to bottom of the endoscope body 3 of the second endoscope 30, as illustrated in FIG. 14, may be adapted to/accommodated, and it may be ensured that each part of the endoscope body 300 may abut against the snapping portion 150 at the corresponding position, thereby improving the connection effect between the connecting device 10 and the second endoscope 30.

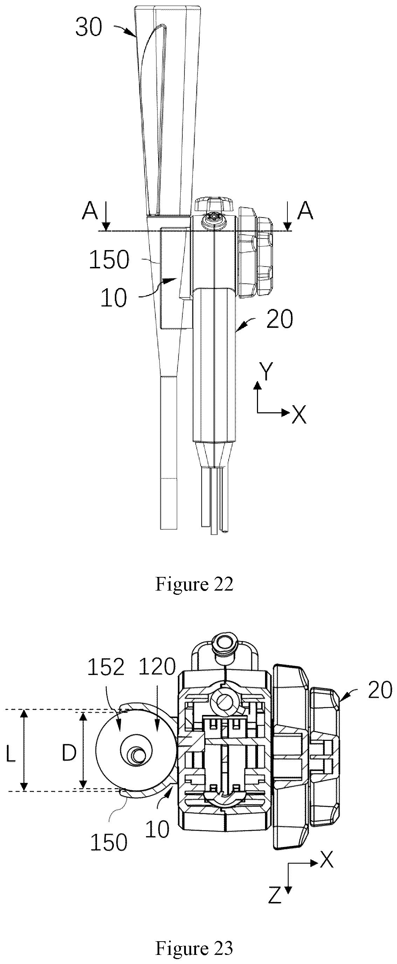

Referring to FIG. 22, in the sixth embodiment, the edge regions on two sides of the inner wall 121 of the receiving groove 120 together form a snapping portion 150, and the receiving groove 120 is formed within the snapping portion 150. It is to be noted that in this embodiment, the side wings 151 are the side edges of the inner wall 121 of the receiving groove 120, that is to say, the side wings 151 are integrated formed on the inner wall 121 of the receiving groove 120 without a clear boundary. The two side edges of the inner wall 121 arranged opposite to each other in the third direction Z are configured to be able to snap/clamp on two sides of the second endoscope 30, so that the second endoscope can be fixed within the receiving groove 120.

Further, referring to FIG. 22 and FIG. 23, the snapping portion 150 forms an opening 152 communicated with the receiving groove 120. The snapping portion 150 is so configured that the second endoscope 30 can be received into the receiving groove 120 via the opening 152. That is to say, an opening 152 communicated with the receiving groove 120 is formed between the two side wings 151 of the snapping portion 150, and the second endoscope 30 can be received into the receiving groove 120 along the first direction X through the opening 152. In some embodiments, the opening has a maximum size D in the third direction Z, and the portion of the second endoscope 30 accommodated within the receiving groove 120 has a maximum size L; it is satisfied that: D≤L. Thus, when the second endoscope 30 is received into the receiving groove 120 via the opening 152, a force will be applied onto the snapping portion 150, causing the two opposite wings 151 thereof to be elastically deformed and get separated apart, so that the second endoscope 30 can be smoothly fitted into the receiving groove 120. This facilitates an installation of the second endoscope 30. When the second endoscope 30 is received within the receiving groove 120, since the maximum size of the opening 152 of the snapping portion 150 is less than or equal to the size of the portion of the second endoscope 30 accommodated within the receiving groove 120, the snapping portion 150 can limit/confine the second endoscope 30 within the receiving groove 120, and prevent it from falling out, thereby ensuring the stability of the connection.

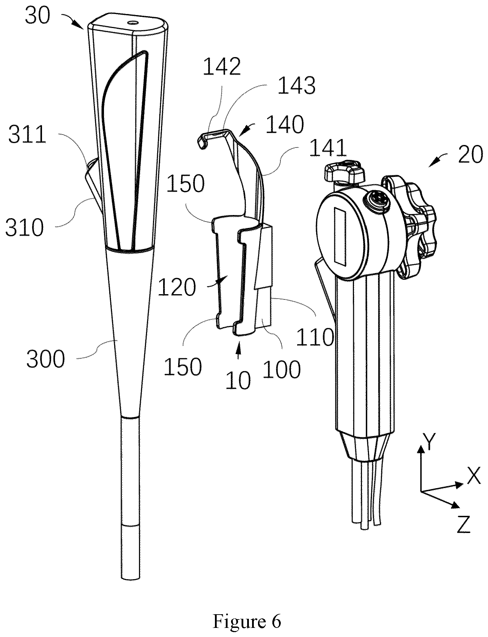

Referring to FIG. 1 and FIG. 6 again, in the first embodiment, the connecting component 130 may also comprise a snapping portion 150, and the configuration as well as position of this snapping portion 150 may be the same as that of the snapping portions 150 in the second embodiment, the third embodiment, and the sixth embodiment. That is to say, the snapping portion 150 can co-operate with the hook portion 140 to jointly couple with the second endoscope 30, so as to achieve multiple connections and improve the stability of the connection. Of course, as described before, the hook portion 140 and the snapping portion 150 may also be provided separately in the connecting device 10 in different embodiments. That is to say, in some embodiments, the connecting component 130 may have a hook portion 140 but do not have a snapping portion 150, and in other embodiments, the connecting component 130 may have a snapping portion 150 but do not have a hook portion 140.

Similarly, referring to FIG. 25 again, in the eighth embodiment, the connecting component 130 may also comprise a snapping portion 150, and the configuration as well as position of this snapping portion 150 may be the same as that of the snapping portions 150 in the second embodiment, the third embodiment, and the sixth embodiment. That is to say, the snapping portion 150 can co-operate with the winding portion 180 to jointly couple with the second endoscope 30, so as to achieve multiple connections and improve the stability of the connection. Of course, as described before, the winding portion 180 and the snapping portion 150 may also be provided separately in the connecting device 10 in different embodiments. That is to say, in some embodiments, the connecting component 130 may have the winding portion 180 but do not have the snapping portion 150, and in other embodiments, the connecting component 130 may have the snapping portion 150 but do not have the winding portion 180.

Referring to FIG. 12 and FIG. 13 again, in some embodiments, the connecting component 130 further comprises a protrusion 160 connected to the body 100 and disposed in the receiving groove 120. The protrusion 160 is arranged to protrude with respect to the inner wall 121 of the receiving groove 120. The protrusion 160 is designed for contacting with the second endoscope 30 when the second endoscope 30 is received in the receiving groove 120. The contact surface between the protrusion 160 and the second endoscope 30 is a rough surface 161. By provision of the protrusion 160 in the receiving groove 120, the protrusion 160 can get into contact with the second endoscope 30 in the receiving groove 120, thus providing a support for the second endoscope 30. By this, the second endoscope 30 may be stably held in the receiving groove 120. The contact surface between the protrusion 160 and the second endoscope 30 is designed as a rough surface 161, thus, a friction force acting on the second endoscope 30 may be increased, thereby preventing the second endoscope 30 from moving in the receiving groove 120, which improves the connection stability further.

Further, in some embodiments, the protrusion 160 is made of elastic materials. Therefore, the protrusion 160 can be elastically deformed to adapt to second endoscopes 30 of different sizes, which prevents an over-pressing of the second endoscope 30; at the same time, the protrusion 160 may fit more snugly on the endoscope body 300 of the second endoscope 30, which improves the stability of the connection.

Further, in some embodiments, multiple protrusions 160 are provided and spaced apart in the second direction Y, wherein the second direction Y is the extension direction of the receiving groove 120. Therefore, the endoscope body 300 of the second endoscope 30 may be supported at different positions along its length direction, which further ensuring a fitting stability of the second endoscope 30 within the receiving groove 120.

In some embodiments, a direction extending from the receiving groove 120 towards the connecting portion 110 is defined the first direction X. The connecting component 130 forms a first orthographic projection on a plane perpendicular to the first direction X, and the body 100 forms a second orthographic projection on this plane. The first orthographic projection falls within the second orthographic projection. The connecting device 10 with such a structure is more small and compact, and the connecting component 130 can be arranged in the receiving groove 120 and does not protrude outside of the body 100, which prevents its spatial interference with the first endoscope 20, the second endoscope 30 or other surgical tools.

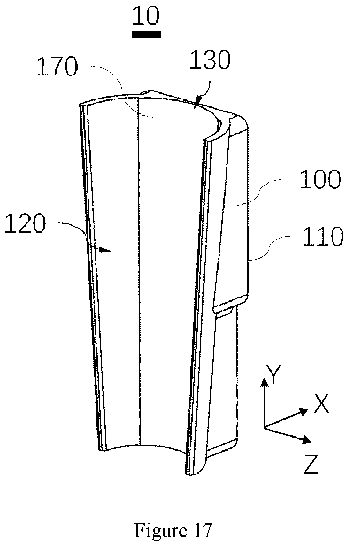

Specifically, Referring to FIGS. 17 to 20, in some embodiments, the connecting component 130 comprises a bonding portion 170 connected to the body 100 and adhered to at least a portion of the inner wall 121 of the placement slot 120. The bonding portion 170 is designed for bonding the second endoscope 30 so that the second endoscope 30 can be fixed within the receiving groove 120. By providing a bonding portion 170 on the inner wall 121 of the receiving groove 120, the second endoscope 30 can be coupled to the inner wall 121 of the receiving groove 120 via the bonding portion 170. By this, on the one hand, the connection stability can be ensured and the second endoscope 30 can be prevented from shaking in the receiving groove 120; on the other hand, the bonding portion 170 does not extend beyond the spatial contour extent of the body 100, so that spatial interferences with other tools during surgery can be avoided. Still further, a double-sided tape can be used for the bonding portion 170, which has a simple structure, is easy to manufacture, and has a low cost.

Further, in some embodiments, multiple bonding portions 170 are provided and spaced apart from each other. Therefore, the second endoscope 30 can be bonded at multiple positions on the inner wall 121 of the receiving groove 120, further ensuring the stability of the second endoscope 30 in the receiving groove 120, and ensuring a connection stability between the connecting device 10 and the second endoscope 30 .

Accordingly, an embodiment of the present disclosure also provides an endoscope assembly. Referring to FIGS. 1 to 20 again, the endoscope assembly comprises the connecting device 10 in the above embodiments, and a first endoscope 20 connected to the connecting portion 110.

It is to be noted that the first endoscope 20 can be any kind of medical endoscope, and can be selectively provided according to actual surgical requirements.

Further, in some embodiments, the connecting device 10 and the first endoscope 20 are formed into an integral structure. That is to say, the connecting device 10 and the first endoscope 20 are integrated, and there may be no obvious boundary/division line therebetween. Any combination location between two portions in the integrated structure can be considered as the connecting portion 110. It can be understood that the connecting portion 110 and the first endoscope 20 are integrated.

Further, in some embodiments, the endoscope assembly further comprises a second endoscope 30. The second endoscope 30 is received in the receiving groove 120 of the connecting device 10, and is connected with the connecting component 130 of the connecting device 10, so as to be fixed within the receiving groove 120.

It is to be noted that the second endoscope 30 can also be any kind of medical endoscope, and can be selectively provided according to actual surgical requirements.

Further, in some embodiments, the second endoscope 30 comprises an endoscope body 300 and a clip entrance 310. The endoscope body 300 is received in the receiving groove 120; the clip entrance 310 is arranged on the endoscope body 300; the hook portion 140 of the connecting component 130 hooks on the clip entrance 310 to connect with the second endoscope 30.

Further, in some embodiments, the second endoscope 30 comprises an endoscope body 300 received within the receiving groove 120 and engaging with the snapping portion 150 of the connecting component 130 in a snapping way.

Further, in some embodiments, the second endoscope 30 comprises an endoscope body 300 received within the receiving groove 120; the bonding portion 170 of the connecting component 130 is bonded with the second endoscope 30, so that the second endoscope 30 can be fixed within the receiving groove 120.

Further, in some embodiments, the second endoscope 30 comprises an endoscope body 300 received within the receiving groove 120; the winding portion 180 of the connecting component 130 is designed for winding around the second endoscope 30 to connect with the second endoscope 30.

In the embodiments described above, the description for each embodiment has its own focus. For parts not described in detail in a certain embodiment, reference may be made to the relevant description of other embodiments. In particular, the endoscope assembly of the embodiments of the present disclosure can comprise all the technical features and technical effects of the connecting device 10 described above, and the corresponding technical features and technical effects are specifically described hereinbefore, which will not be repeated here.

The connecting device and endoscope assembly provided in the embodiments of the present disclosure are introduced in detail above, and the principles and implementations of the present disclosure are elaborated by specific examples. The description of the above embodiments is only intended to be helpful for understanding the technical solution and core idea of the present disclosure. Those ordinary skills in the art should understand that they can still modify the technical solutions described in the aforementioned embodiments, or replace some of the technical features therein with equivalents. These modifications or replacements will not cause the essence of the corresponding technical solutions to deviate from the scope of the technical solution of each of the embodiments of the present disclosure.

## Claims

1. A connecting device (10) for connecting a first endoscope (20) and a second endoscope (30), comprising:
a body (100) comprising a connecting portion (110) for connecting the first endoscope (20), wherein the body (100) is further provided with a receiving groove (120) that is configured to face away from the connecting portion (110) to receive the second endoscope (30);
a connecting component (130) provided on the body (100), wherein the connecting component (130) is configured to connect the second endoscope (30) so that the second endoscope (30) may be fixed in the receiving groove (120).

2. The connecting device (10) according to claim 1,
wherein a direction extending from the receiving groove (120) towards the connecting portion (110) is defined as a first direction (X), an extending direction of the receiving groove (120) is defined as a second direction (Y), with the first direction (X) intersecting with the second direction (Y);
wherein the connecting component (130) forms a first orthographic projection on a plane perpendicular to the first direction X, and the body (100) forms a second orthographic projection on this plane; wherein at least a portion of the first orthographic projection falls outside the second orthographic projection, and is located further away in the second direction (Y) with respect to the second orthographic projection.

3. The connecting device (10) according to claim 1, wherein the connecting component (130) comprises:
a hook portion (140) connected to the body (100), wherein at least a portion of the hook portion (140) is located further away in the second direction (Y) with respect to the body (100), and the second direction (Y) is defined as the extension direction of the receiving groove (120);
the hook portion (140) is configured to hook on the second endoscope (30) for connecting with the second endoscope (30).

4. The connecting device (10) according to claim 3, wherein the second endoscope (30) comprises an endoscope body (300) and a clip entrance (310) connected with each other, wherein the clip entrance (310) protrudes from the endoscope body (300), and the hook portion (140) is configured to at least partially hook on the clip entrance (310).

5. The connecting device (10) according to claim 4, wherein the hook portion (140) is configured to wind around the clip entrance (310).

6. The connecting device (10) according to claim 4, wherein the hook portion (140) comprises:
an extension arm (141) connected to the body (100), wherein at least a portion of the extension arm (141) is located on a side of the body (100) that extends further away in the second direction (Y);
a clamping lug (142) which is opposite to and spaced apart from the extension arm (141);
an abutting arm (143) which is connected between the extension arm (141) and the clamping lug (142);
wherein the abutting arm (143) is configured for contacting with a top surface (311) of the clip entrance (310), and the extension arm (141) and the clamping lug (142) are respectively configured for contacting with two opposite entrance side faces (312) of the clip entrance (310), such that the hook portion (140) may hook on the clip entrance (310); wherein the two entrance side faces (312) are respectively connected to the top surface (311) of the clip entrance.

7. The connecting device (10) according to claim 6, wherein the extension arm (141) comprises:
a first surface (1411) that extends away from the connecting portion (110) in the first direction (X), and is configured for abutting against a side surface of the endoscope body (300) that faces towards the body (100);
a second surface (1412) that is connected to the first surface (1411) at an end away from the body (100), and is configured for abutting against a side face of the clip entrance (310) that faces towards the body (100);
wherein the first direction (X) is a direction extending from the receiving groove (120) towards the connecting portion (110).

8. The connecting device (10) according to claim 1, wherein the connecting component (130) comprises:
a winding portion (180) connected to the body (100), wherein at least a portion of the winding portion (180) is provided at one side of the body (100) that extends further away in the second direction (Y), said second direction (Y) being an extension direction of the receiving groove (120);
wherein the winding portion (180) is configured for winding around the second endoscope (30) so as to connect with the second endoscope (30).

9. The connecting device (10) according to claim 1, wherein the direction extending from the receiving groove (120) towards the connecting portion (110) is defined as a first direction (X), the extension direction of the receiving groove (120) is defined as a second direction (Y), and the body (100) has a third direction (Z), the first direction (X), the second direction (Y) and the third direction (Z) intersecting with each other;
the connecting component (130) forms a first orthographic projection on a plane perpendicular to the first direction (X), the body (100) forms a second orthographic projection on this plane, wherein at least parts of the first orthographic projection fall outside the second orthographic projection, and are symmetrically distributed at two sides of the second orthographic projection with respect to the third direction (Z).

10. The connecting device (10) according to claim 1, wherein the connecting component (130) comprises:
a snapping portion (150) that is connected on one side of the body (100) opposite to the connecting portion (110) and is provided on both sides of the receiving groove (120) with respect to a third direction (Z); a direction extending from the receiving groove (120) to the connecting portion (110) is defined as a first direction (X), an extending direction of the receiving groove (120) is defined as the second direction (Y), with the first direction (X), the second direction (Y) and the third direction (Z) intersecting with each other;
wherein the snapping portion (150) is configured to clamp on both sides of the second endoscope (30) such that the second endoscope (30) may be fixed within the receiving groove (120).

11. The connecting device (10) according to claim 10, wherein the snapping portion (150) forms an opening (152) communicated with the receiving groove (120), and the snapping portion (150) is so configured that the second endoscope (30) may be received into the receiving groove (120) via the opening (152);
wherein the opening (152) has a maximum dimension D in the third direction (Z), and a portion of the second endoscope (30) that is accommodated within the receiving groove (120) has a maximum size L, wherein D≤L.

12. The connecting device (10) according to claim 10, wherein the snapping portion (150) comprises:
at least two side wings (151) that are oppositely arranged with respect to the third direction (Z), wherein the at least two side wings (151) are connected to the body (100) and are respectively provided at two side edges of the receiving groove (120) with respect to the third direction (Z).

13. The connecting device (10) according to claim 12, wherein the side wing (151) comprises:
a main body (1511) that connected to the body (100) and is provided with a fitting slot (1514); and
a rotating body (1512) that is rotatably connected to the main body (1511) and is arranged in the fitting slot (1514),
wherein the rotating body (1512) is configured for contacting the second endoscopes (30) in a rotatable way to guide the second endoscope (30) into the receiving groove (120).

14. The connecting device (10) according to claim 13, wherein the rotating body (1512) is provided with a plurality of anti-slip recesses (1513), wherein the plurality of anti-slip recesses (1513) are spaced apart along an outer circumferential surface of the rotating body (1512).

15. The connecting device (10) according to claim 13, wherein the rotating body (1512) is made of elastic materials.

16. The connecting device (10) according to any one of claims 12-15, wherein a plurality of the snapping portions (150) are provided and are spaced apart in the second direction (Y).

17. The connecting device (10) according to claim 16, wherein a minimum spacing between at least two side wings (151) in one snapping portions (150) is different from a minimum spacing between at least two side wings (151) in another snapping portion (150).

18. The connecting device (10) according to claim 1, wherein the connecting component (130) comprises:
a protrusion (160) that is connected to the body (100) and is arranged in the receiving groove (120), wherein the protrusion (160) is configured to protrude with respect to an inner wall (121) of the receiving groove (120);
wherein the protrusion (160) is configured to contact with the second endoscope (30) when the second endoscope (30) is received within the receiving groove (120), wherein a contact surface between the protrusion (160) and the second endoscope (30) is a rough surface (161).

19. The connecting device (10) according to claim 18, wherein the protrusion (160) is made of elastic materials.

20. The connecting device (10) according to claim 18, wherein a plurality of protrusions (160) are provided in a spaced manner in the second direction (Y), wherein the second direction (Y) is the extension direction of the receiving groove (120).

21. The connecting device (10) according to claim 1, wherein a direction extending from the receiving groove (120) towards the connecting portion (110) is defined as a first direction (X);
wherein the connecting component (130) forms a first orthographic projection on a plane perpendicular to the first direction (X), the body (100) forms a second orthographic projection on the plane, and the first orthographic projection falls within the second orthographic projection.

22. The connecting device (10) according to claim 1, wherein the connecting component (130) comprises:
a bonding portion (170) that is connected to the body (100) and is adhered to at least a portion of an inner wall (121) of the receiving groove (120);
the bonding portion (170) is configured for bonding the second endoscope (30) so that the second endoscope (30) may be fixed within the receiving groove (120).

23. The connecting device (10) according to claim 22, wherein a plurality of bonding portions (170) spaced apart from each other is provided.

24. The connecting device (10) according to claim 1, wherein the body (100) and the connecting component (130) are of an integrally formed structure.

25. An endoscope assembly, comprising:
the connecting device (10) according to any one of claims 1 to 24; and,
a first endoscope (20) connected to the connecting portion (110).

26. The endoscope assembly according to claim 25, wherein the connecting device (10) and the first endoscope (20) are of an integrally formed structure.

27. The endoscope assembly according to claim 25, further comprising:
a second endoscope (30) that is received in the receiving groove (120) of the connecting device (10), and is connected with the connecting component (130) of the connecting device (10) so as to be fixed within the receiving groove (120).

28. The endoscope assembly according to claim 27, wherein the second endoscope (30) comprises:
an endoscope body (300) that is received within the receiving groove (120);
a clip entrance (310) provided on the endoscope body (300);
wherein a hook portion (140) of the connecting component (130) hooks on the clip entrance (310) to connect with the second endoscope (30).

29. The endoscope assembly according to claim 27, wherein the second endoscope (30) comprises:
an endoscope body (300) that is received within the receiving groove (120) and engages with the snapping portion (150) of the connecting component (130) in a snapping way.

30. The endoscope assembly according to claim 27, wherein the second endoscope (30) comprises:
an endoscope body (300) received in the receiving groove (120);
wherein a bonding portion (170) of the connecting component (130) is bonded to the second endoscope (30) so that the second endoscope (30) can be fixed within the receiving groove (120).

31. The endoscope assembly according to claim 27, wherein the second endoscope (30) comprises:
an endoscope body (300) received in the receiving groove (120);
wherein a winding portion (180) of the connecting component (130) is configured to wind around the second endoscope (30) so as to be connected to the second endoscope (30).
